(19) Europäisches Patentamt
European Patent Office
Office européen des brevets

(11) **EP 2 495 356 A1**

(12) **EUROPEAN PATENT APPLICATION**

(43) Date of publication:
**05.09.2012  Bulletin 2012/36**

(21) Application number: **12157418.0**

(22) Date of filing: **29.02.2012**

(51) Int Cl.:
*C25D 11/26* (2006.01)  *C01G 23/08* (2006.01)
*B82Y 30/00* (2011.01)  *A61L 27/30* (2006.01)
*C25D 11/18* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.03.2011  IT MI20110312**

(71) Applicant: **Dental Tech S.r.l.**
**20020 Misinto, Milano (IT)**

(72) Inventors:
• **CAVALLET, Gabriele**
**I-20813 Bovisio Masciago, MONZA E BRIANZA (IT)**

• **CAVALLET, Graziano**
**I-20823 Lentate sul Seveso, MONZA E BRIANZA (IT)**
• **FERRARI, Franco**
**I-21047 Saronno, VARESE (IT)**
• **CARINCI, Francesco**
**I-40124 BOLOGNA (IT)**
• **CRISTINO, Vito**
**I-44100 FERRARA (IT)**
• **CARAMORI, Stefano**
**I-44123 FERRARA (IT)**
• **BIGNOZZI, Carlo Alberto**
**I-44121 FERRARA (IT)**

(74) Representative: **Long, Giorgio et al**
**Jacobacci & Partners S.p.A.**
**Via Senato 8**
**20121 Milano (IT)**

(54) **Dental implant  with nanostructured surface and process for obtaining it**

(57)     The present inventionprovides materials and methods for preparing nanotubes made of titanium dioxide as coating for osseointegrated biomedical prostheses, in particular for dental implants. Such coatings, made with electrochemical and chemical methods, have the purpose of promoting osseointegration of endosseous implants.

In particular, the invention concerns a method for preparing an implant device made of titanium or alloys thereof, comprising the following steps:
a) subjecting said implant device made of titanium or alloys thereof to an anodizing process, in water or in an organic solvent containing an amount of water lower than 25% by volume, so as to form a layer of nanotubes made of titanium dioxide on said implant device;
b) processing said implant device at a temperature higher than 450˚C or higher than 500˚C for a period of time sufficient to transform said titanium dioxide into its anatase allotropic crystalline form;
c) loading said nanotubes made of titanium dioxide in anatase allotropic crystalline form with ions of a substance having antimicrobial activity.

The invention also describes an implant device, specifically a dental implant screw, able to be obtained with the method of the invention.

**Description**

BACKGROUND OF THE INVENTION

Field of the invention

The present invention provides materials and methods for preparing nanotubes based on titanium dioxide as coating for osteointegrated biomedical prostheses, in particular for dental implants. Such coatings, made with electrochemical and chemical methods, have the purpose of promoting osteointegration of endosseous implants.

State of the art

Titanium and its alloys are materials widely used in implant technology thanks to their mechanical and biocompatibility characteristics.

When exposed to oxygen, titanium tends to oxidise, increasing its characteristics that promote osteointegration. Among the allotropic forms of titanium dioxide particularly used in implant technology, there is anatase, which is generally prepared as a colloidal suspension used to coat screws and implants. Such a coating is indeed able to modulate the genic expression and the translational processes of osteoblasts and it has an extremely low bacterial colonization potential.

Osteointegration is a process by which the implanted material causes an activation of the bone tissue in which it is inserted, such as to determine an adhesion of the bone tissue to the implant. The osteointegration process is extremely complex and not entirely understood. In it there is intervention by immunogenic control mechanisms (self-recognition) typical of all processes that foresee the use of transplants, mechanisms of osteoconduction (process by which the implanted material represents a support for new bone production) and osteoinduction (process by which the implanted material causes one or more molecular signals that induce new bone synthesis). The osteointegration process can be considered to be complete in about 60 days, corresponding to the consolidation time of fractures.

In terms of endosseous implants, an important role is performed by the effect of the material from which they are made and by the design of the implant itself. Indeed, both factors have various degrees of influence on the osteointegration process.

Titanium currently represents the material of first choice for dental and orthopaedic prostheses since it combines excellent mechanical properties with high osteointegration.

With regard to the design of the implant, the following are normally taken into consideration: (1) a macrodesign, (2) a minidesign, (3) a microdesign and (4) a nanodesign.

The first is the macroscopic design of the implant. With reference to dental implants, there are implants of various shapes, for example cylindrical or conical.

The second is given by the characteristics of the design of the spirals, by the shape of the corners of the spirals (which can be bevelled or sharp) and it is in the order of millimetres.

The third is given by the characteristics of the surface that, for example, can be smooth or rough. For this purpose there are various methods that cause differences in the size of the micropores that are created at the surface of the implant.

Finally, we can talk of nanodesign: this corresponds to the molecular organisation on the surface of the implant. Currently, implants are made of titanium that in air oxidises into titanium dioxide with a stochastic distribution of the two crystalline forms, rutile and anatase. Preliminary studies found in the literature demonstrate the possibility of producing surfaces completely coated with anatase, which enhances the osteointegration properties of titanium.

Various authors agree that the nano-roughness produced on the surface of the implants plays a fundamental role in determining their osteointegration. A review on the subject was published by A. Wennerberg, T. Abrektsson, The Int. Journal of Oral & Maxillofacial Implants pg. 63-74 Vol. 25, No. 1, 2010, where the analysis of the nano-roughness of the most widely used implants (TiUnite, SLActive, Nanotite or Ossospeed) in all cases reveals the absence of regular nanometric structures and the presence of surfaces with variable roughness.

In addition to these surface characteristics, there are other extremely important ones for osteointegration. Some studies, indeed, have demonstrated that by coating the surfaces of the implants with "titanium nanotubes" biocompatibility is substantially increased and that such cellular affinity is closely correlated to the diameter of the nanostructures, especially in the proliferation of osteoblasts and in the mineralization processes. In these studies the nano-tubular structures are made through electrochemical oxidation processes in aqueous solution, in the presence of acetic or phosphoric acid and hydrofluoric acid with a system having three electrodes that operates with potentiostatic control.

Summary of the invention

An object is to provide implant devices made from titanium that combine an antimicrobial activity with osteointegration properties, thus contributing to preventing infection phenomena, which are often the cause of rejection of the implant.

A further object of the invention are dental implants, typically implant screws, equipped with high osteointegration and preferably with antimicrobial activity.

A further object of the invention is to provide a process for making titanium dioxide nanotubes loaded with a molecule having antimicrobial activity on an implant device.

The problem forming the basis of the present invention is therefore that of obtaining the aforementioned objects.

Such a problem is solved by implant devices and by a process for producing them, as outlined in the attached claims, the definitions of which form an integral part of the present description.

Further characteristics and advantages of the invention will become clearer from the description of some example embodiments, given hereafter for indicating and not limiting purposes.

Brief description of the figures

Figure 1 schematically represents an anodization device for the formation of nanotubes on titanium;

- figure 2 shows the typical curve of electric currents recorded during the anodization processes in different solvents DMSO, NMF or $H_2O$;
- figure 3 represents an SEM image of a titanium surface that has $TiO_2$ nanotubes made via anodization in water;
- figure 4 represents an SEM image of a titanium surface that has $TiO_2$ nanotubes made via anodization in DMSO;
- figure 5 shows a graph with the comparison between genes expressed at 15 days by the cells cultivated on the surfaces with nanotubes and the same genes derived from cells cultivated on smoothed titanium;
- figure 6 shows a graph with the comparison between genes expressed at 30 days by the cells cultivated on the surfaces with nanotubes and the same genes derived from cells cultivated on smoothed titanium.

Detailed description of the invention

The invention consists of making nanotubular structures of titanium dioxide on endosseous implants made from titanium or metal alloys containing titanium through anodization processes with a simple feed that operates with two electrode systems in solvents of the organic or aqueous type, in particular in dimethyl sulphoxide (DMSO), N-methylformamide (NMF) and in water ($H_2O$ and in the production of surface structures that promote the osteointegration process. Inside the nanotubular structure ionic substances with antimicrobial activity like monovalent silver ions ($Ag^+$) are then inserted, preferably adsorbed on nanoparticles of titanium dioxide having dimensions 2-20 nm that carry out antimicrobial activity.

Nano-tubular porous nano-structures equipped with high active surface have been obtained on implant devices made from titanium by using electrochemical anodic dissolution technology, a process able to produce nanotubular structures, consisting of titanium dioxide, of variable length of between 100 nanometres and 3 microns.

The use of this technology ensures that the nanotubular structures are an integral part of the metal, thus allowing high strength due to the direct interconnection with the underlying metal. The anodizations can be carried out in different solvents of organic nature, like for example DMSO (dimethyl sulphoxide) or NMF (N-methylformamide) or in aqueous solvent, using specific electrolytic compositions specified hereafter.

Based on the selected solvent and the anodization time it is possible to obtain nanotubes characterised by a different diameter and length.

The anodization is carried out by imposing a potential difference between the endosseous implant (anode) and a counter-electrode (cathode) consisting of titanium or platinum. In this invention the anodization procedures have been carried out using a two electrode configuration and using a direct current voltage-adjustable power supply available on the market at low cost. The potentials applied in the anodization processes can vary between 20 and 50 V based on the solvent used and based on the thickness of the anodised substrate required.

The choice of the solvent is important in determining the anodization times necessary to obtain the desired nanostructure. In particular, by operating with aqueous solvents it is possible to obtain the formation of nanotubes in times of the order of 6-12 hours, whereas in the organic solvents used (DMSO or NMF) such times are at least double (12-24 hours). In general, it is possible to operate with anozidation times varying between 6 and 48 hours, depending on the solvent used and the length of the nanotubes that it is wished to obtain. The use of longer anodization times entails the formation of nanotubes of greater length that can reach 3 microns and diameters within the range 50-100 nm.

It should be noted that the presence of water in the organic solvents used is essential for the formation of the titanium dioxide constituting the nanotubular structure. Therefore, when the anodization process is carried out in a solution of an organic solvent, in particular DMSO or NMF, such a solution will contain water in the range from 5% to 20% by volume.

The application of a potential difference between the endosseous implant and the counter-electrode produces, in the initial stage, a layer of compact oxide that leads to the passivation of the implant. Such passivation results in a decrease in the electric current that passes through the electrolytic cell. The presence of fluorides in the electrolytic solution is essential in order to obtain the dissolution of the layer of oxide previously formed and the consequent formation of nanotubular structures.

The dissolution process of the layer of oxide that leads to the formation of the nanotubular structure is indicated by the following equation:

$$\mathrm{TiO_2 + 6F^- + 4H^+ = [TiF_6]^{2-} + 2H_2O}$$

where the hexafluoride titanium ion is soluble in the solvent that makes up the electrolytic solution. As the source of fluorides it is possible to use different salts at different concentration levels, like for example KF (0.1 M), $NH_4F$ (0.5% p/p) or HF (2% v/v).

The anodization speed is an important parameter in order to obtain nanotubes of the desired dimensions. Preferably, an electric current density of between 10 and 100 $mA/cm^2$ will be used.

After the anodization process, the implant device is subjected to heating at temperatures of over 450°C, typically over 500°C, for example about 550°C, for a sufficient time to convert the titanium dioxide nanotubes into the crystalline allotropic form of anatase, compact its surface and eliminate possible organic impurities present on the device. Preferably, the heating lasts for a time of between 30 minutes and 2 hours, for example about 1 hour.

After the transformation of the titanium dioxide into anatase, the implant device is loaded with ionic species having antimicrobial activity.

For this purpose the implant device of the invention is immersed in a suspension of titanium dioxide nanoparticles of a size in the range 2-30 nm in a solution of 50% ethyl alcohol by volume in water, containing silver ions with concentrations in the range $10^{-4}$-$10^{-1}$ M.

Such solutions can be prepared using different salts or complex silver ions like for example silver nitrate, silver acetate, silver citrate, silver lactate, silver sulphadiazine, etc..

The titanium dioxide nanoparticles are able to absorb the $Ag^+$ ions at their surface and they have dimensions such as to allow their insertion inside nanotubular structures with diameters within the range 30-50 nm. The implant device is immersed in such solutions and subjected to sonication for times of the order of 5-15 minutes.

An object of the present invention is therefore a method for preparing an implant device that comprises the following steps:

a) subjecting said implant device made of titanium or alloys thereof to an anodizing process as described above, in water or in an organic solvent containing an amount of water lower than 25% by volume, said organic solvent preferably being DMSO or NMF, so as to form a layer of nanotubes made of titanium dioxide on said implant device;
b) processing said implant device at a temperature higher than 450°C or higher than 500°C for a period of time sufficient to transform said titanium dioxide into its anatase allotropic crystalline form;
c) loading said nanotubes made of titanium dioxide in anatase allotropic crystalline form with ions of a substance having antimicrobial activity, preferably $Ag^+$ ions, in which said ions are previously adsorbed on nanoparticles of titanium dioxide having a dimension allowing them to be inserted in said nanotubes.

A further object of the invention consists of an implant device made from titanium or alloys thereof comprising an outer layer of titanium dioxide nanotubes in anatase allotropic crystalline form, characterised in that said titanium dioxide nanotubes are loaded with titanium dioxide nanoparticles on which an ionic substance having antimicrobial activity is adsorbed. Preferably, said ionic substance consists of $Ag^+$ ions. Yet Another object of the invention consists of a dental implant, typically a screw, made from titanium or alloys thereof, characterised in that it comprises a layer of titanium dioxide nanotubes in anatase allotropic crystalline form on its surface.

Preferably, said nanotubes are loaded with an ionic substance having antimicrobial activity, such as $Ag^+$ ions. In a particularly preferred embodiment, said nanotubes are loaded with titanium dioxide nanoparticles on which said ionic substance having antimicrobial activity is adsorbed.

PREPARATION METHODS

The preparation methods described hereafter apply both to dental implants in the form of screws and more generally to endosseous implants of different shape and size. The methods have been devised using foils of titanium or of titanium alloys with a thickness of a few hundreds of microns and an area of 2.5x5 cm, pretreated through a sandblasting process to obtain macroporous surfaces analogous to those used for endosseous implants. The choice of operating with titanium foils is linked to the need to conduct the biological analyses described hereafter, which require a particular geometry for the samples investigated. Before carrying out the anodic dissolution, which leads to the formation of the nanotubular structures, the titanium foils were previously cleaned with washing in water and acetone and sonicated with ultrasound in an aqueous solution of detergent (Alconox) for a time of about 10 minutes.

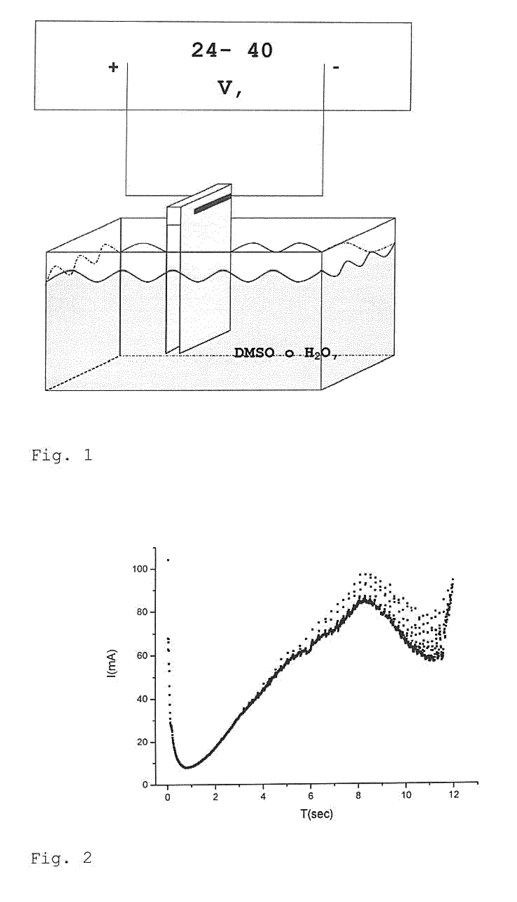

The anodizations were carried out by frontally placing two titanium dioxide foils in an electrolytic solution at a distance of about 6 mm (Fig. 1). The electrolytic solution was kept under stirring through a magnetic stirrer. The area of the electrode immersed in the electrolytic solution was about $2.5 \times 2.5$ cm. The remaining non-immersed surface was used for the electric contact and to prevent the rise of electrolytic solution by capillary action leading to short circuits of the cell.

The anodizations were carried out in three solvents: $H_2O$ DMSO and NMF, obtaining analogous results for the organic solvents DMSO and NMF.

In the case of the aqueous solvent, the anodizations were carried out by applying, to the two titanium foils, a potential difference of 24V for a time of between 6 and 12h (Fig. 2) in a solution containing KF (0.1 M) as fluoride provider and $K_2SO_4$ (0.5 M) as support electrolyte. The non-linear curve of the current recorded during the anodizations, as shown in Fig. 2, is due to the competition between the oxide formation and dissolution processes. If the oxide formation speed is higher than its dissolution there is passivation of the electrode with a consequent decrease in the current recorded. Vice-versa, when the dissolution is faster than the oxidation process of the titanium there is a larger surface of the metal exposed to the solution, which means an increase in the current recorded. The formation of ordered structures of nanotubes is due to the dissolution of the oxide through "preferential paths" dictated by the reinforcing of the electric field in the areas in which the initial dissolution made the layer of oxide thinner, thus speeding up the growth and the relative dissolution of the oxide in that particular area. The speed with which the dissolution occurs is essential - dissolutions that are too fast or slow do not lead to the formation of oxide.

In terms of the anodic dissolution carried out in DMSO or NMF, containing HF 2% by volume or $NH_4F$ at 0.5% by weight, a potential difference of 40V was applied for a time of between 12 and 24h. The anodizations were carried out at room temperature (about 20°C).

The presence of fluorides is essential to excavate the surfaces of oxide formed following the anodization process of the titanium foil. In Figure 2 it can be seen that during anodization there is an increase in current due to a higher dissolution speed of the layer of oxide with respect to its formation, whereas the decrease in current is due to a passivation of the electrode caused by the formation of a thick layer of oxide. The choice of the anodization time is decisive for the purposes of obtaining a particular morphology at the surface of an endosseous implant. High anodization times in general lead to the formation of structures of nanotubes that can reach lengths of the order of 3 microns, whereas anodization times that are too short do not lead to the formation of nanotubular structures.

Once the anodization process has concluded, the foils were treated with water and acetone and sonicated with ultrasound for about 10 minutes in distilled water to eliminate possible residues of titanium dioxide not stably bonded to their surface. Finally, the anodized titanium foils were subjected to heating at 550°C for 1h, in order to convert the titanium dioxide nanotubes into the anatase allotropic form, compact its surface and eliminate possible organic impurities present on the foils.

The current produced during the anodization was recorded, at time intervals of one minute, by a Velleman DVM 1200 digital multimeter interfaced with a PC or every thirty seconds with a Tenma 72-7730 digital multimeter, also interfaced with the PC. The anodizations were carried out thanks to a Thurlby Thandar Instruments EX752M power supply.

The anodized titanium samples according to the procedures described above were immersed in a suspension of titanium dioxide nanoparticles of a size in the range 2-30 nm in a solution of 50% ethyl alcohol by volume in water, containing silver ions with concentrations in the range $10^{-4}$-10-$^1$ M.

Such solutions can be prepared using different salts or complex silver ions like for example silver nitrate, silver acetate, silver citrate, silver lactate, silver sulphadiazine, etc..

The titanium dioxide nanoparticles are able to adsorb the $Ag^+$ ions at their surface and their dimensions allow their insertion inside nanotubular structures with diameters within the range 30-50 nm. The samples made from anodized titanium were immersed in the suspensions containing silver ions and subjected to sonication for times of the order of 5-15 minutes. The samples were then washed with ethanol and dried at 60°C.

MORPHOLOGICAL CHARACTERISATION

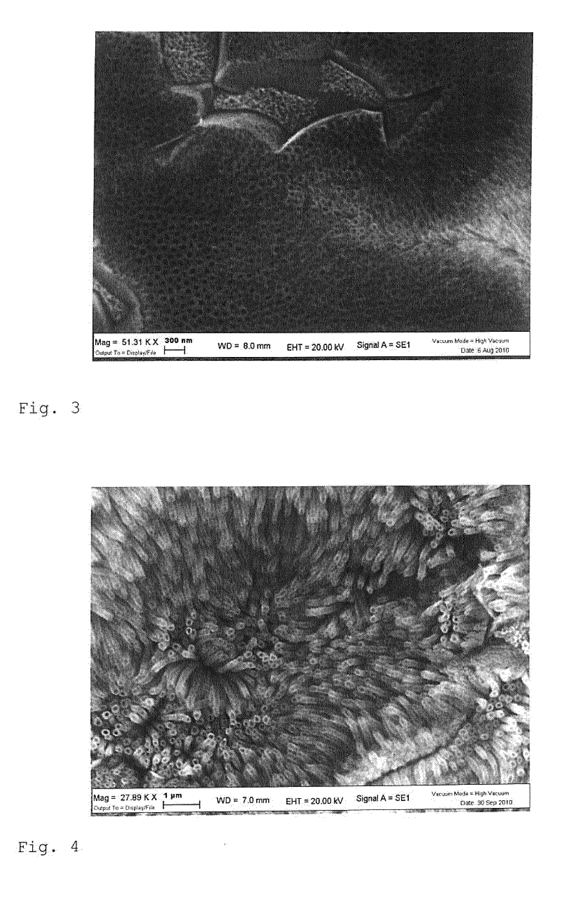

The treated titanium foils were characterised through SEM electron microscopy showing that, both for anodic dissolutions carried out in water (Fig. 3) and for those carried out in DMSO or NMF (Fig. 4), there is formation of nanotubular structures, present in a homogeneous manner on the entire electrode, both on the face arranged at the front of the counter-electrode and on the opposite face. From the SEM images it is possible to see how the nanotubes produced by anodization in water are about 300-400 nm in length whereas those made in DMSO or NMF reach lengths of one micron.

ANTIMICROBIAL ACTIVITY

Verification was carried out of the antimicrobial activity of the titanium foils, prepared according to the procedures described earlier that foresee the anodization process and the subsequent insertion of silver ions, carrying out tests with the following bacterial strains: *Escherichia coli, Staphyloccoccus aureus, Pseudomonas aeruginosa* and *Candida albicans.*

EXPERIMENTAL PROCEDURE

Microorganisms

The following test strains were used:

| | | |
|---|---|---|
| *Pseudomonas aeruginosa* | *ATCC* | *15442* |
| *Staphyloccoccus aureus* | *ATCC* | *6538* |
| *Escherichia coli* | *ATCC* | *10536* |
| *Candida albicans* | *ATCC* | *10231* |

The strains come from the Department of Experimental and Diagnostic Medicine, Microbiology Section, of the University of Ferrara and they were acquired from the companies Diagnostic International Distribution SpA and VWR International Srl.

Storage

The bacterial strains were stored frozen in culture broth and glycerol at 50% (v/v); before use they were transplanted on TSA slants and kept in a refrigerator at 4°C ± 2°C.
*Candida albicans* was stored frozen in culture broth and glycerol at 50% (v/v); before use they were transplanted on Malt Extract Agar slants and kept in a refrigerator at 4°C ± 2°C.

CULTURE MEDIA AND REACTANTS

Media

Tryptone Soya Agar (TSA) for the bacterial strains and Malt Extract Agar (MEA) for *Candida albicans*

Diluent

Triptone,

| | | |
|---|---|---|
| casein pancreatic digestion | 1.0 g | OXOID |
| NaCl | 8.5 g | MERCK |

Distilled water as needed up to 1000 ml

APPARATUSES

| | |
|---|---|
| - stove for dry sterilization | KW |
| - Steam autoclave | COLUSSI |
| - Thermostat | MEMMERI |
| - Vortex agitator | VELP |
| - Chronometer | ARBORE |
| - Micropipette | GILSON |

Preparation of the test suspensions

The bacterial strains and the strain of *Candida albicans,* once unfrozen, were transplanted twice in succession on TSA slants (MAE for *Candida albicans*) and incubated at 37˚C $\pm$ 1˚C for 18 hours, obtaining the working culture. Within 2 hours from the start of the test the working culture was suspended in diluents using glass balls and the suspension was diluted until a count of between $1.5 \times 10^7$ and $5.0 \times 10^7$ ufc/ml was obtained (test suspension).

Counting of the test suspensions

Serial dilutions of the test suspensions having a concentration of between $1.5 \times 10^7$ and $5.0 \times 10^7$ ufc/ml were carried out in diluents up to $10^{-6}$ - $10^{-7}$ . Double counting was carried out for inclusion in Agar. The number of ufc/ml of the suspension was determined after an incubation period of 24 hours at 37˚C $\pm$ 1˚C.

Performing the test

For every strain of the microorganisms a test tube containing 5 ml of test suspension having a concentration of between $1.5 \times 10^7$ and $5.0 \times 10^7$ ufc/ml was prepared.

The titanium foils treated according to the anodization and silver ion insertion procedures and the untreated ones (control) were immersed in the test suspensions containing the difference microbe species and extracted after 10 seconds. The samples were left under a sterile hood in contact with air for a time equal to 5 minutes. At the end of this time, the titanium foils were placed in Petri dishes and covered with liquid culture medium, kept at a temperature of 45˚C. The Petri dishes were placed under stirring for 1 minute, in order to promote homogeneous diffusion over the entire dish of the microorganisms stuck to the surface of the titanium foils and the medium was then left to solidify. The Petri dishes were finally placed in an incubation cell at 37˚C for 24 hours.

Once this time had passed, the dishes were examined evaluating the development of the bacterial and fungus colonies.

RESULTS

The analysis of the dishes relative to the control samples highlighted the growth of an extremely large number of microbe colonies whereas the dishes relative to the foils treated with the procedures described in this patent application demonstrated antimicrobial activity, inducing the total mortality of the microbe cells that came into contact with its surface. Compared to an uncountable number of colonies (more than tens of thousands) formed in the Petri dishes relative to the controls, in the Petri dishes including the treated foils there was no colony growth.

BIOLOGICAL CHARACTERISATION RELATIVE TO THE OSTEOINDUCTION PROCESSES

Principle of the investigation

The purpose of this investigation is to verify the biological effects of a titanium alloy whose surface has been coated with nanotubes.

For this purpose stem cell cultures were prepared deriving from dental pulp selected as clone CD105 positive, CD90 positive, CD73 positive and CD 34 negative, which when placed in culture with appropriate medium differentiate into osteoblasts [Pittenger MF et al. Multilineage potential of adult human mesenchymal stem cells. Science 1999; 284: 143-147.]. Such cells were cultivated on titanium discs coated with nanotubes and on machined titanium discs (i.e. not coated with nanotubes) and used as control. After 15 and 30 days the cells were detached, the messenger RNA was extracted and the expression of specific genes whose function is correlated to osteodifferentiation was measured, through Real Time PCR. All of this is to verify the osteo-differentiation potential of the surface of the nanotubes. Indeed, when the metallic prostheses are inserted in the bones of the patients they do not only stimulate an osteoblast activity, but also an activity of the stem cells that is relevant for the osteointegration process.

Materials and Methods

*Isolation of stem cells from dental pulp*

The dental pulp from which the stem cells is to be obtained was extracted from third molar teeth of healthy individuals of ages comprised between 20 and 25 years after gaining informed consent.

The pulp was then immersed in a digestion solution (3 mg/ml of collagenase type I and 4 mg/ml of dispase dissolved in 4 ml of a saline solution 0.1 M of phosphate buffer saline (PBS) integrated with 100 U/ml of penicillin, 100 mg/ml of streptomycin, and 500 mg/ml of clarithromycin) and incubated for 1 hour at 37˚C. Once digested, the solution was filtered with 70 micron Falcon cellular filters (Sigma Aldrich).

After filtration, the cells were placed in T75 flasks, containing culture medium $\alpha$-MEM integrated with 20% FBS, 100 $\mu$M ascorbic acid, 2 mM L-Glutamine, 100 U/ml penicillin, 100 mg/ml streptomycin and incubated at 37˚ C and 5% $CO_2$. The medium was changed twice per week. Shortly before the cells merged, they were divided into new flasks.

For the test, the stem cells in the second step were detached through trypsin and seeded on plates whose surface has been coated with titanium nanotubes. Another series of wells containing the cells seeded on machined titanium plates were used as controls.

The cells were collected 15 and 30 days after treatment for the extraction of RNA.

RNA extraction

The reverse transcription to cDNA was performed directly by the lysate of the cells placed in culture through the "TaqMan Gene Expression Celle-to-Ct" kit (Ambion Inc., Austin, Tx, USA), following the manufacturer's instructions. In short, the cells were lysate with a lysis buffer and the RNA freed in this solution was retrotranscribed to cDNA using a specific retrotranscriptase and relative buffer.

The cDNA thus obtained was amplified through Real Time PCR using an amplification mixer, the TaqMan Gene Expression Master Mix provided with the kit and specific amplification tests (primers and probes) for the genes to be investigated.

Real Time PCR

The expression of the genes investigated was quantified through Real Time PCR.

The gene expression levels were normalised through a housekeeping reference gene RPL13A. Thereafter, the expression of the cells treated with the nanotubes was compared with that of the untreated cells, through the statistical method of $\Delta\Delta$Ct [Livak and Schmittgen, 2001].

The forward and reverse primers and the probes for the selected genes were designed using the Primer Software Express programme (Applied Biosystems, Foster City, CA, USA). This experiment used Taq Man probes (Applied Biosystems, Foster City, CA, USA) of genes whose expression is linked to osteoinduction and osteodifferentiation.

All of the PCR reactions were carried out in a volume of 20 microlitres using the instrument ABI PRISM 7500 (Applied Biosystems, Foster City, CA, USA), following a PCR protocol that foresees an initial denaturing of the DNA at 95˚C followed by two steps, a denaturing at 95˚C for 15 sec and an annealing of the primers and probes at 60˚C for 1 minute, for 40 cycles.

RESULTS

In order to test the osteoinducing effect of the titanium nanotubes, the expression levels of those genes whose function is correlated to the mineralization and deposition of the bone matrix were measured, in stem cells subjected to treatment for 15 and 30 days, the most relevant time period for the purposes of the osteointegration process.

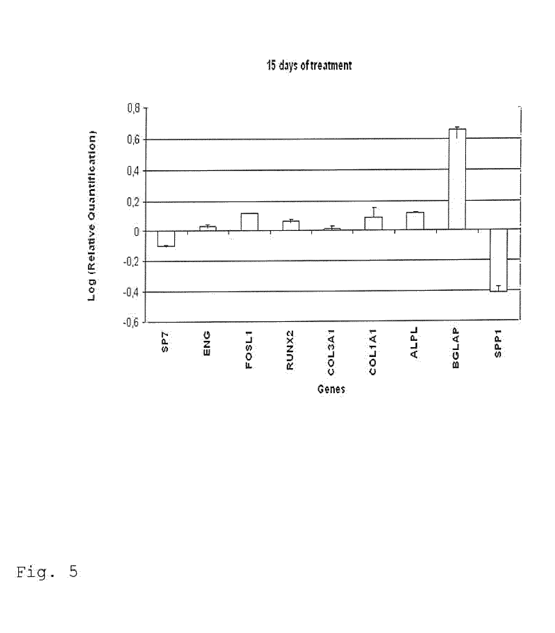

Through Real Time PCR analysis was carried out of two essential transcription factors in osteoblast differentiation (RUNX2 and FOSL1), some genes linked to the differentiation of the bone SPP1 (Osteopontin), BGLAP (osteocalcin) and ALPL (alkaline phosphatase), the genes of collagen (COL1A1 and COL3A1) and a characteristic gene of stem cells the expression of which varies during cellular differentiation (ENG). After 15 days of treatment in the stem cells cultivated on the titanium nanotubes with respect to the control there is a marked over-expression of the genes RUNX2, FOSL1, ALPL, BGLAP and COL1A1 and a moderate over-regulation of the genes ENG and COL3A1. On the other hand the genes SP7 and SPP1 are under-expressed (Fig. 5).

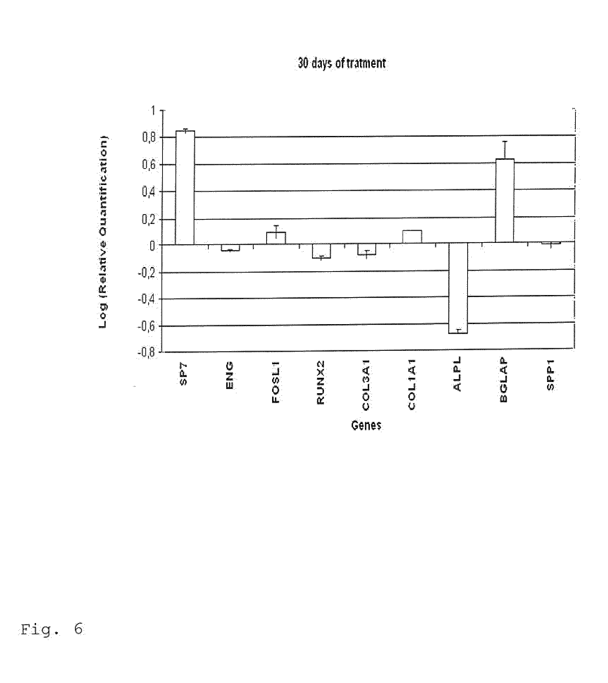

The results of the real time PCR demonstrated that after 30 days of treatment, with respect to the control cells, the osteogens SP7, FOSL1, COL1A1 and BGLAP are over-expressed whereas RUNX2, ENG, ALPL and COL3A1 are under-regulated (Fig. 6). The expression of SPP1 substantially does not change.

By analysing the results in greater detail, we can see that the transcription factor RUNX2 is over-expressed in the treated cells with respect to the control cells after 15 days and under-expressed after 30 days. RUNX2 is an important modulator of the differentiation of osteoblasts that is activated in the first steps of differentiation and performs an essential role in the maturing of the osteoblasts and in homeostasis. It can be seen that the titanium nanotubes thus promote osteodifferentiation of the stem cells in the first treatment steps. An opposite effect was observed for another transcription factor, SP7, which regulates the formation of bones and the differentiation of the osteoblasts and that is downstream of RUNX2.

In treated stem cells SP7 is under-expressed in the first days of treatment but then tends to increase in the next 30 days.

The titanium nanotubes are also able to modulate the expression of the genes that encode for the proteins collagen of the extracellular matrix as collagen of type $1\alpha1$ (COL1A1) and collagen of type $3\alpha1$ (COL3A1). After 15 days of treatment it was observed that there was a marked over-expression of COL1A1 and a more modest over-regulation of COL3A1.

At 30 days of treatment the expression of COL3A1 falls, because the titanium nanotubes induce the synthesis of the matrix in the first stages of differentiation and proliferation of the osteoblasts.

It has been demonstrated that the synthesis of collagen of type I is associated with the differentiation of the osteoblasts in the initial step, followed by the synthesis of ALP and osteocalcin (BGLAP).

In such a stage ALPL is significantly over-expressed after 15 days of treatment. The increased expression of ALPL was widely associated with the differentiation of the osteoblasts.

Osteocalcin (BGLAP), a protein involved in the mineralization and bone reabsorption, is also over-expressed after 15 and 30 days treatment.

Osteocalcin is activated immediately before mineralization and its expression continues to increase in the subsequent steps.

The titanium nanotubes then determine the significant over-expression of the gene FOSL1. Such a gene encodes for a component of the transcription factor AP-1 the sites of which are present in the osteogenes promoters, including alkaline phosphatase, collagen I, osteocalcin.

Finally, the expression of endoglin (ENG), a surface marker used to characterise mesenchymal stem cells, remains unchanged in the first 15 days of treatment with the titanium nanotubes. Thereafter, its expression falls substantially in the treated stem cells, indicating the osteoinducing effect of this biomaterial on stem cells. Indeed, it has been demonstrated that the disappearance of the antigen ENG during osteogenesis suggests that this protein is involved in the regulation of osteogenesis.

CONCLUSIONS

The treatment of implant devices made of titanium or alloys thereof through anodization and subsequent insertion of silver ions has demonstrated that the titanium nano-tubular nanostructures carry out antimicrobial activity and strongly influence the behaviour of stem cells in vitro, increasing their proliferation, differentiation in osteoblasts and deposition of the bone matrix.

**Claims**

1. Method for preparing an implant device made of titanium or alloys thereof, comprising the following steps:

    a) subjecting said implant device made of titanium or alloys thereof to an anodizing process, in water or in an organic solvent containing an amount of water lower than 25% by volume, so as to form a layer of nanotubes made of titanium dioxide on said implant device;
    b) processing said implant device at a temperature higher than 450˚C or higher than 500˚C for a period of time sufficient to transform said titanium dioxide into its anatase allotropic crystalline form;
    c) loading said nanotubes made of titanium dioxide in anatase allotropic crystalline form with ions of a substance having antimicrobial activity.

2. Method according to claim 1, wherein said ions of a substance having antimicrobial activity are $Ag^+$ ions.

3. Method according to claim 1 or 2, wherein said ions of a substance having antimicrobial activity are previously adsorbed on nanoparticles of titanium dioxide having a dimension allowing them to be inserted in said nanotubes.

4. Method according to claim 3, wherein said titanium dioxide particles have a dimension ranging from 2 to 20 nm.

5. Method according to any one of claims 1 to 4, wherein said organic solvent is selected between dimethylsulfoxide and N-methylformamide.

6. Method according to any one of claims 1 to 5, wherein said anodizing step a) is carried out by employing an arrangement made of two-electrodes and an adjustable current voltage power supply, operating at potential difference ranging from 20 to 50 V.

7. Method according to any one of claims 1 to 6, wherein anodizing times range from 6 to 48 hours.

8. Method according to claim 7, wherein the times for anodization in aqueous solvent are in the range of 6-12 hours, whereas the times for anodization in organic solvents are in the range of 12-24 hours.

9. Method according to any one of claims 1 to 8, wherein, when the anodization is carried out in an organic solvent, said organic solvent contains an amount of water ranging from 5% to 20% by volume.

10. Method according to any one of claims 1 to 9, wherein said anodizing step a) is carried out in the presence of fluoride salts.

11. Method according to claim 10, wherein said fluoride salts are selected from among KF 0.1 M, $NH_4F$ 0.5% w/w and HF 2% v/v.

12. Method according to any one of claims 1 to 11, wherein said anodizing step a) is carried out with a current density ranging from 10 to 100 $mA/cm^2$.

13. Method according to any one of claims 1 to 12, wherein said step b) is carried out for a time period ranging from 30 minutes to 2 hours.

14. Method according to any one of claims 1 to 13, wherein said step b) is carried out at a temperature of about 550°C and for a time period of about an hour.

15. Method according to any one of claims 1 to 14, wherein said step c) is carried out by submerging said implant device in a suspension of nanoparticles of titanium dioxide having a size in the range of 2-30 nm in a 50% by volume aqueous solution of ethyl alcohol, containing silver ions with a concentration ranging from $10^{-4}$ to $10^{-1}$ M, in which the silver ions are preferably derived from a salt selected among silver nitrate, silver acetate, silver citrate, silver lactate, silver sulfadiazine, and subjecting the implant device in said solution to sonication for times ranging from 5 to 15 minutes.

16. Implant device made of titanium or alloys thereof comprising an outer layer of nanotubes made of titanium dioxide in the anatase allotropic crystalline form, **characterised in that** said nanotubes made of titanium dioxide are filled with nanoparticles of titanium dioxide on which a ionic substance having antimicrobial activity is absorbed.

17. Implant device according to claim 16, wherein said ionic substance having antimicrobial activity consists of $Ag^+$ ions.

18. Implant device according to claim 16 or 17, wherein said ionic substance is adsorbed on titanium dioxide particles having a size allowing them to be inserted in said nanotubes.

19. Implant device according to any one of claims 16 to 18, wherein said nanotubes have a length ranging from 100 nm to 3 microns and diameters ranging from 30 to 50 nm and wherein said titanium dioxide particles which can be inserted in said nanotubes have a size ranging from 2 to 30 nm.

20. Implant device according to any one of claims 16 to 19, wherein said device is a dental implant screw.

Fig. 1

Fig. 2

Fig. 3

Fig. 4

Fig. 5

Fig. 6

Europäisches
Patentamt

European
Patent Office

Office européen
des brevets

# EUROPEAN SEARCH REPORT

Application Number

EP 12 15 7418

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | POPAT ET AL: "Decreased Staphylococcus epidermis adhesion and increased osteoblast functionality on antibiotic-loaded titania nanotubes", BIOMATERIALS, ELSEVIER SCIENCE PUBLISHERS BV., BARKING, GB, vol. 28, no. 32, 24 August 2007 (2007-08-24), pages 4880-4888, XP022211120, ISSN: 0142-9612, DOI: 10.1016/J.BIOMATERIALS.2007.07.037 | 1,2,5,6, 10-14 | INV.<br>C25D11/26<br>C01G23/08<br>B82Y30/00<br>A61L27/30<br><br>ADD.<br>C25D11/18 |
| A | * abstract *<br>* page 4881, left-hand column, last paragraph - right-hand column, paragraph first *<br>* page 4881, paragraph 2.1.-2.2. *<br>----- | 3,4,7-9, 15-20 | |
| A | US 2009/093881 A1 (BANDYOPADHYAY AMIT [US] ET AL) 9 April 2009 (2009-04-09)<br><br>* abstract *<br>* paragraphs [0005], [0010] - [0012], [0024], [0028], [0038], [0039] *<br>* paragraphs [0045] - [0050]; examples 1,2 *<br>----- | 2-4,6, 10,11, 16-20 | |
| | | | TECHNICAL FIELDS SEARCHED (IPC)<br><br>C25D<br>C01G<br>B82Y<br>A61L |
| A | US 2010/136325 A1 (REDDY GANTA S [US] ET AL) 3 June 2010 (2010-06-03)<br>* abstract *<br>* paragraphs [0014], [0017], [0060], [0065], [0071], [0074], [0076] *<br>----- | 2-4, 16-20 | |
| A | US 2008/110342 A1 (ENSOR DAVID S [US] ET AL) 15 May 2008 (2008-05-15)<br>* paragraph [0198] *<br>----- | 2-4, 16-20 | |

The present search report has been drawn up for all claims

| Place of search | Date of completion of the search | Examiner |
|---|---|---|
| Munich | 4 June 2012 | Haering, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 12 15 7418

This annex lists the patent family members relating to the patent documents cited in the above-mentioned European search report.
The members are as contained in the European Patent Office EDP file on
The European Patent Office is in no way liable for these particulars which are merely given for the purpose of information.

04-06-2012

| Patent document cited in search report | Publication date | Patent family member(s) | Publication date |
|---|---|---|---|
| US 2009093881 A1 | 09-04-2009 | NONE | |
| US 2010136325 A1 | 03-06-2010 | EP 2089480 A2<br>US 2010136325 A1<br>WO 2008136866 A2 | 19-08-2009<br>03-06-2010<br>13-11-2008 |
| US 2008110342 A1 | 15-05-2008 | CN 101534954 A<br>DE 112007002725 T5<br>GB 2455948 A<br>JP 2010509056 A<br>KR 20090082418 A<br>US 2008110342 A1<br>US 2010031617 A1<br>WO 2008063870 A1 | 16-09-2009<br>24-09-2009<br>01-07-2009<br>25-03-2010<br>30-07-2009<br>15-05-2008<br>11-02-2010<br>29-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. WENNERBERG ; T. ABREKTSSON.** *The Int. Journal of Oral & Maxillofacial Implants,* 2010, vol. 25 (1), 63-74

- **PITTENGER MF et al.** Multilineage potential of adult human mesenchymal stem cells. *Science,* 1999, vol. 284, 143-147